Europäisches Patentamt

European Patent Office

Office européen des brevets

(11) Publication number: **0 093 518**
**B1**

(12) **EUROPEAN PATENT SPECIFICATION**

(45) Date of publication of patent specification: **14.08.85**

(21) Application number: **83302063.9**

(22) Date of filing: **13.04.83**

(51) Int. Cl.⁴: **B 01 J 23/86,** B 01 J 23/78, C 07 C 15/46, C 07 C 5/333

(54) Catalysts for para-ethyltoluene dehydrogenation.

(30) Priority: **27.04.82 US 372268**

(43) Date of publication of application:
**09.11.83 Bulletin 83/45**

(45) Publication of the grant of the patent:
**14.08.85 Bulletin 85/33**

(84) Designated Contracting States:
**BE DE FR GB IT NL**

(56) References cited:
**FR-A-1 156 171**
**GB-A- 832 297**
**US-A-2 683 180**
**US-A-3 364 277**

(73) Proprietor: **MOBIL OIL CORPORATION**
**150 East 42nd Street**
**New York New York 10017 (US)**

(72) Inventor: **Chu, Chin-Chiun**
**6 Nathan Drive**
**New Brunswick New Jersey 08902 (US)**
Inventor: **Burress, George Thomas**
**80 Claire Drive**
**Bridgewater New Jersey 08807 (US)**

(74) Representative: **West, Alan Harry**
**Mobil Court 3 Clements Inn**
**London WC2A 2EB (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

## Description

This invention relates to improved catalysts for the selective dehydrogenation of dialkyl aromatic hydrocarbons to produce alkyl vinyl aromatic hydrocarbons, more particularly to catalysts for the production of para-methylstyrene (PMS) via the dehydrogenation of para-ethyltoluene (PET).

The vinyl benzenes play a particularly important role in the preparation of synthetic plastics and resins. The polymerization of styrenes, for example, to produce polystyrene resins is well known.

Styrene and styrene derivatives are typically produced from ethyl benzene materials by dehydrogenation over solid catalysts in the presence of co-fed steam, and at temperatures ranging from 500° to 700°C. The catalysts found to be the most effective for this process are those which are based on potassium oxide (carbonate) promoted, chromium oxide stabilized, iron oxide material. Catalysts of this type are said to be self-regenerative inasmuch as, in addition to their effectiveness in promoting dehydrogenation, they also promote the water gas reaction in the presence of the steam co-feed, to thereby remove coke which would otherwise build up on and deactivate the catalyst The lifetime of such self-regenerative catalysts is thus determined by the effectiveness of the catalyst in maintaining its activity for conversion of ethylbenzene materials such as para-ethyltoluene for any given steam/hydrocarbon ratio in the feed. Catalysts of this type which can maintain such activity at generally lower steam/hydrocarbon ratios are, of course, more economically desirable.

Considerable research has been directed toward attempts to improve the activity and selectively of this class of catalysts. For a given steam to hydrocarbon ratio in the feed, any improvement which results in either increasing the selectivity (moles of desired product per mole of reactant reacted) or the conversion (moles of reactant reacted per mole of starting material) without lowering the other is economically attractive since the result is that the yield (moles of desired product produced per mole of reactant) of the product has been increased. Any increase in the numerical value of the yield results in a more efficient operation with more reactant being converted into the desired product. In commercial operations, many of which produce millions of pounds of product per year, a trade-off is frequently effected between selectivity and conversion. An increase of only 1 or 2 percentage points in the selectivity can result in a substantial savings of starting materials. An increase in conversion can substantially reduce capital expenditure and energy consumption. The trade-off may vary depending on raw materials costs, energy costs, and the age of the plant.

Attempts have been made to improve the conversion effectiveness and selectivity of iron oxide type dehydrogenation catalysts for use in various alkylaromatic dehydrogenation reactions.

Riesser; U.S. Patent 4,152,300; issued May 1, 1979, for example, discloses that an improvement in ethylbenzene dehydrogenation catalyst selectivity can be realized by incorporating small amounts of certain metal oxide materials into dehydrogenation catalyst compositions comprising mixtures of iron oxide, potassium oxide, vanadium oxide and, optionally, chromium oxide.

Courty; U.S. Patent 4,134,858; issued January 19, 1979, discloses an iron oxide based dehydrogenation catalyst containing particular amounts of clay to improve the conversion, selectivity and yield of styrene and divinylbenzes produced by dehydrogenation of ethyl- or diethylbenzene.

Notwithstanding such attempts to improve iron oxide based dehydrogenation catalysts, there is a continuing need to formulate catalysts of this type which can be used to realize improved conversion, selectivity, and/or yield in the dehydrogenation of other types of alkylaromatic materials such as, for example, in the production of para-methylstyrene from para-ethyltoluene.

Accordingly it is an object of the present invention to provide an improved iron oxide based dehydrogenation catalyst especially useful for the dehydrogenation of para-ethyltoluene to produce para-methylstyrene.

It is a further object of the present invention to provide a para-ethyltoluene dehydrogenation process employing a catalyst which provides a significant increase in para-ethyltoluene conversion with little or no corresponding drop in para-methylstyrene selectivity.

The present invention relates to improved dehydrogenation catalyst compositions especially useful for the selective dehydrogenation of para-ethyltoluene to produce para-methylstyrene. Such a catalyst comprises from about 30% to 60% by weight of an iron oxide component, calculated as ferric oxide, from about 13% to 48% by weight of a potassium compound component, calculated as potassium oxide, and from 0% to about 5% by weight of a chromium compound component, calculated as chromic oxide. At least 50% by weight of the active iron oxide component is present in the form of particles ranging in size from about 0.05 to 0.5 micron.

The present invention also relates to a dehydrogenation process wherein para-ethyltoluene, preferably along with steam, is passed over the foregoing catalyst composition at a temperature from about 500°C to 700°C with a LHSV of from about 0.3 to 2.0 hour$^{-1}$, to produce para-methylstyrene.

The dehydrogenation catalyst compositions of the present invention contain as an essential catalytic component one or more iron compounds, generally in the form of iron oxide. Many forms of iron oxide can be used in the catalyst compositions of this invention. Typically, iron oxides employed in catalyst preparations of this sort are a synthetically produced, powdered red, red-brown, yellow or black pigment. The red or red-brown pigments are highly pure ferric oxide, while the black pigment is the magnetic form, ferrosoferric oxide ($Fe_3O_4$), which is usually found in the catalyst under various reaction conditions. The yellow iron oxides consist of the monohydrated form of ferric oxide. These oxides are prepared by various methods, e.g., oxidation of iron compounds, roasting, precipitation, calcination, etc. A suitable form of iron compound is the monohydrated yellow iron oxide used in the preparation of catalysts according to U.S. Patent Numbers 3,360,597, issued December 26, 1967, and 3,364,277; issued January 16, 1968. Particularly suitable are pigment grade red iron oxides of purities exceeding 98% weight. These red oxides have surface areas ranging from 2 to 50 $m^2$/gram and are commercially available in particle sizes of from 0.1 to 2 microns. The iron compound is present in the catalyst in either one or a mixture of both of its possible oxidation states, i.e., as ferrous iron or ferric iron or mixtures thereof, as for example, ferrosoferric iron.

The catalyst compositions herein generally comprise from about 30% to 60% by weight, preferably from about 35% to 55% by weight, of iron oxide calculated as ferric oxide. It has been surprisingly discovered that the size of the iron oxide particles used to formulate the catalyst composites of the present invention can affect the activity and selectivity of the resulting dehydrogenation catalysts. In accordance with the present invention, at least 50% by weight, and preferably at least 90% by weight, of the iron oxide component utilized is in the form of particles of from about 0.05 to 0.5 micron in size, more preferably from about 0.1 to 0.4 micron in size. By maintaining such particle size restrictions on the active iron oxide component, catalysts can be realized which provide unexpectedly high conversion of saturated hydrocarbons such as para-ethyltoluene to unsaturated products. With respect to dehydrogenation of para-ethyltoluene, such catalysts are also unexpectedly selective to production of para-methylstyrene. Improvements in activity and selectivity of such catalysts can thus permit utilization of lower amounts of co-fed steam in carrying out the selective dehydrogenation reaction.

The dehydrogenation catalyst compositions of the present invention also essentially comprise, as a catalyst promoter, one or more potassium compounds. The potassium promoter material can be added to the catalyst in various forms. For example, it may be added as the oxide, or as other compounds which are convertible, at least in part, under calcination conditions, to the oxides, such as the hydroxides, the carbonates, the bicarbonates, the phosphates, the borates, the acetates, and the like. A particularly preferred potassium compound is potassium carbonate. The potassium compound is generally present in the catalyst as a potassium oxide, a potassium carbonate or a mixture thereof. High carbon dioxide partial pressures in the reaction gases will favor high carbonate to oxide ratios and vice versa within the potassium component.

The catalyst compositions herein generally comprise from about 13% to 48% by weight, and preferably from about 27% to 41% by weight, of potassium promoter compound, calculated as potassium oxide. When the potassium compound actually used in formulating the catalysts is the preferred potassium carbonate, the catalyst compositions herein generally can contain from about 20% to 70% by weight, and preferably from about 40% to 60% by weight, of potassium compound, calculated as potassium carbonate. Preferably the molar ratio of potassium compound, as $K_2O$ or $K_2CO_3$, to iron compound, as $Fe_2O_3$, ranges from about 0.8:1 to 1.5:1, more preferably from about 0.9:1 to 1.3:1.

An optional, but highly preferred, component of the present catalyst composition is a chromium compound which serves as a stabilizer for the active catalytic components. Chromium compounds have, in fact, typically been added to alkali-promoted iron oxide catalysts to extend their life. Chromium, when optionally used in the compositions of this invention, can be added to the catalyst in the form of a chromium oxide or in the form of chromium compounds which decompose upon calcination to chromium oxides, as for example, chromium nitrates, hydroxides, acetates, and the like. Chromium can also be added in the form of alkali metal chromates. If potassium chromates are used, such materials can, of course, also contribute to the requisite concentration of potassium compound essentially present in the dehydrogenation catalyst compositions as hereinbefore discussed. Thus, the catalyst compositions herein can comprise from 0% to about 5% by weight, and preferably from about 2% to 4% by weight chromium compound, calculated as chromic oxide.

The physical strength of the catalyst composition of the present invention can be improved, if desired, by adding any of a variety of optional binding agents. Binding agents can include, for example, calcium aluminate and portland cement. The density of the catalyst compositions herein can likewise be modified by the addition of various filler substances, for example, combustible materials such as sawdust, carbon, wood flour, etc. Such materials can be added to the compositions during preparation and thereafter burned out after the catalyst pellets have been formed. Other porosity promoting aids include graphite and aqueous solutions of methylcelluose, which also facilitate extrusion of catalyst pellets as hereinafter described. If employed, binders and other fillers generally can comprise up to about 10% by weight of the catalyst composition.

The catalyst compositions of the present invention are in general prepared by admixing the essential and desired optional components as hereinbefore described with water and by thereafter drying and optionally calcining the resulting mixture. Drying temperatures can range from about 50°C to 500°C. Calcination temperatures can range from about 300°C to 650°C, preferably from about 450°C to 550°C.

The components of the catalyst compositions herein can be admixed in various ways. One method comprises ballmilling together a mixture of the desired oxides and/or compounds decomposable upon calcination to oxides, adding a small amount of water, and extruding the paste formed to produce small pellets, which are then dried and calcined. Another method is to dissolve the components together, spray dry these components to form a resulting powder, calcine the powder into the resultant oxides, and then add sufficient water to form a paste which is extruded into pellets, dried and calcined. Another procedure involves precipitating those materials which are precipitatable, such as iron and chromium, as the resultant hydroxides, partially dewatering the resultant precipitate, adding soluble salts of the other required metals, and then subsequently extruding, drying and calcining the resulting pellets. A preferred method involves dry-blend powdering of oxides and/or compounds decomposable upon calcination to the oxides, adding water, optionally containing dissolved therein soluble compounds decomposable upon calcination to the oxides, then mixing and/or mulling the resultant paste, pelletizing the mixture, subsequently substantially drying at a temperature from about 50°C to about 300°C, followed by calcining the pellets at a temperature ranging from about 450°C to about 500°C, to form the final product. The drying and calcining could be carried out stepwise in the same furnace by suitable programming of the furnace temperature. Alternatively, water-insoluble dry powders of oxides and/or compounds decomposable upon calcination to the oxides are dry-mixed, and the balance of the other materials needed are dissolved in water and the resultant solution is used to form the paste with the dry powders. There are many variations of the mixing of dry powders, water and water soluble compounds that give equivalent results and fall within the scope of this invention.

The optimum size of the pellets produced will vary according to the needs of various processes in which they are to be utilized. Cylindrical catalyst pellets having a diameter of from 1.6 to 4.8 mm (1/16 to 3/16 of an inch), and from 3.2 to 12.7 mm (1/8 to 1/2 of an inch) in length are typical. The smaller diameter catalysts are generally more active but provide increased pressure drops.

The catalysts of the present invention are especially effective in promoting the dehydrogenation of para-ethyltoluene to produce para-methylstyrene. Such a dehydrogenation reaction is usually carried out at reaction temperatures of about 500°C—700°C. However, higher or lower temperatures may be used without departing from the scope of this invention. The use of atmospheric, sub-atmospheric, or superatmospheric pressure is suitable. However, it is preferable to operate at as low a pressure as is feasible, and atmospheric or sub-atmospheric pressure is preferred. The process of the invention may be carried out in batch, semi-continuous, or continuous operation, with continuous operation being preferred. The catalyst is employed in the form of a fixed bed, or in fluidized or suspended form. It is preferable to utilize a fixed bed. The reaction may be carried out in single stage reactors or by staging in series reactors. The reactors may be of various designs, e.g., downflow reactors, radial reactors, etc.

With the use of the catalyst of this invention, it is desirable to add steam to the reactant feed to aid in the removal of carbonaceous residues from the catalyst. The weight ratio of steam to hydrocarbon in the reactant feed can generally vary from about 1.3:1 to 4:1. Good results can be obtained using steam to hydrocarbon ratios of from about 1.4:1 to 3.5:1. Catalysts having higher potassium contents generally permit utilization of desirably lower steam to hydrocarbon ratios in the reactant feed.

The contact time of the reactant gas with the catalyst is usually defined in terms of liquid-hourly-space velocity (volume of liquid hydrocarbon reactant at room temperature per volume of catalyst per hour, i.e., LHSV). The LHSV according to this invention may vary from about 0.3 to 2.0 hours$^{-1}$ and is preferably adjusted within this range to effect the degree of conversion desired for the particular feed in question.

The catalysts of the present invention and their use will be further described by the following examples which are provided for illustration and are not to be construed as limiting the invention. It should be noted that advantages resulting from increases of selectivity and/or conversion of only one or two percentage points are extremely significant in a commercial process which may produce many hundreds of thousand pounds of product a day. Catalysts with higher activities, which can result in lower operating temperatures can be significant in lowering costs of plant operations.

Examples I—IV

Dehydrogenation catalysts are formulated by admixing the following materials in the following concentrations:

| Component | Concentration (wt%) |
|---|---|
| $Fe_2O_3$ | 46.0 |
| $K_2CO_3$ | 51.0 |
| $Cr_2O_3$ | 3.0 |
| | 100.0% |

To this mixture is added water to the extent of 10% by weight and the resulting mixture is formed into a paste. The paste is extruded into 3.2 mm (1/8″) pellets which are then dried for several hours at approximately 150°C.

Four catalyst samples of this type are prepared in this manner. Each sample is formulated from iron oxide of a different particle size as shown hereinafter in Table I. The dehydrogenation catalyst samples so prepared are used to dehydrogenate para-ethyltoluene (PET) to form para-methylstyrene (PMS) in a tubular reactor to which PET and steam are introduced. Operating conditions for such dehydrogenation reactions are given as follows:

| Temperature | 620°C |
|---|---|
| Pressure: | Atmospheric |
| PET-LHSV: | $1.0 \text{ Hr.}^{-1}$ |
| Water-LHSV | $1.7 \text{ Hr.}^{-1}$ |

After several hundred hours on stream, the conversion results obtained from each of the four catalyst samples are shown in Table I.

Table I

Dehydrogenation of para-ethyltoluene over dehydrogenation catalysts comprising iron oxide of varying particle size

| Catalyst sample No. | Average particle size of $Fe_2O_3$ (microns) | Conversion of para-ethyl-toluene (mole %) | Selectivity to Para-methyl-styrene (mole %) | Yield of para-methyl-styrene (mole %) |
|---|---|---|---|---|
| 1 | 1—2 | 57.3 | 90.3 | 51.7 |
| 2 | 0.3—0.5 | 58.2 | 89.5 | 52.1 |
| 3 | 0.1—0.2 | 64.1 | 89.0 | 57.0 |
| 4 | 0.1 | 59.0 | 91.5 | 54.0 |

The Table I data indicate that utilization of an $Fe_2O_3$ particle size of about 0.1 to 0.5 microns provides dehydrogenation catalysts having higher activity for conversion of p-ethyltoluene without significant offsetting loss of selectivity to the production of the desired para-methylstyrene dehydrogenation product.

**Claims**

1. A dehydrogenation catalyst composition especially suitable for the dehydrogenation of para-ethyltoluene to selectively form para-methylstyrene, said catalyst composition comprising:

a) from about 30% to 60% by weight of iron oxide, calculated as ferric oxide;

b) from about 13% to 48% by weight of potassium compound, calculated as potassium oxide; and

c) from 0% to about 5% of chromium compound, calculated as chromic oxide;

at least 50% by weight of said iron oxide being present in said composition in the form of particles ranging in size from about 0.05 to 0.5 micron.

2. A composition in accordance with Claim 1 wherein

a) the iron oxide comprises from about 35% to 55% by weight of the composition;

b) the potassium compound comprises from about 27% to 41% by weight of the composition; and

c) the chromium compound comprises from about 2% to 4% by weight of the composition.

3. A composition in accordance with Claim 2 wherein at least 90% of said iron oxide component is present in the form of particles ranging in size from about 0.1 to 0.4 micron.

4. A composition in accordance with any of Claims 1 to 3 wherein the potassium compound used to formulate the catalyst is potassium carbonate comprising from about 20% to 70% by weight of the composition and wherein the molar ratio of $K_2CO_3$ to $Fe_2O_3$ ranges from about 0.8:1 to 1.5:1.

5. A composition in accordance with any of Claims 1 to 4 which additionally contains up to about 10% by weight of a binder/filler component selected from the group consisting of portland cement, calcium aluminate, sawdust, carbon, wood flour, graphite, methylcellulose and mixtures thereof.

6. A process for the dehydrogenation of para-ethyltoluene to selectively form para-methylstyrene, said process comprising contacting para-ethyltoluene under dehydrogenation reaction conditions with a catalyst composition comprising:

a) from about 30% to 60% by weight of iron oxide, calculated as ferric oxide;

b) from about 13% to 48% by weight of potassium compound, calculated as potassium oxide; and

c) from 0% to about 5% of chromium compound, calculated as chromic oxide;

at least 50% by weight of said iron oxide being present in said composition in the form of particles ranging in size from about 0.05 to 0.5 micron.

7. A process in accordance with Claim 6 wherein said dehydrogenation conditions include a temperature of from about 500°C to 700°C, and a liquid hourly space velocity of from about 0.3 to 2.0 hours$^{-1}$.

8. A process in accordance with Claim 6 or 7 wherein para-ethyltoluene is contacted with said catalyst in the presence of steam and wherein the weight ratio of steam to hydrocarbon in the reactant feed ranges from about 1.3:1 to 4:1.

9. A process in accordance with any of Claims 6 to 8 wherein the potassium compound used to formulate said catalyst composition is potassium carbonate comprising from about 20% to 70% by weight of the composition and wherein the molar ratio of $K_2CO_3$ to $Fe_2O_3$ in said composition ranges from about 0.8:1 to 1.5:1.

10. A process in accordance with any of Claims 6 to 9 wherein said catalyst composition aditionally contains up to about 10% by weight of a binder/filler component selected from the group consisting of portland cement, calcium aluminate, sawdust, carbon, wood flour, graphite, methylcellulose and mixtures thereof.

## Patentansprüche

1. Eine Dehydrierkatalysator-Zusammensetzung, die besonders für die Dehydrierung von p-Ethyltoluol unter selektiver Bildung von p-Methylstyrol geeignet ist, wobei die genannte Katalysator-Zusammensetzung umfaßt:

a) von etwa 30 bis etwa 60 Gew.-% Eisenoxid, berechnet als Eisen(III)oxid;

b) von etwa 13 bis 48 Gew.-% einer Kaliumverbindung, berechnet als Kaliumoxid; und

c) von 0% bis etwa 5% einer Chromverbindung, berechnet als Chrom(III)oxid;

wobei wenigstens 50 Gew.-% des genannten Eisenoxids in der genannten Zusammensetzung in Form von Teilchen vorliegen, die in ihrer Größe im Bereich von etwa 0,05 bis 0,5 µm liegen.

2. Zusammensetzung nach Anspruch 1, bei der

a) das Eisenoxid von etwa 35 bis 55 Gew.-% der Zusammensetzung bildet;

b) die Kaliumverbindung von etwa 27 bis 41 Gew.-% der Zusammensetzung bildet; und

c) die Chromverbindung von etwa 2 bis Gew.-% der Zusammensetzung bildet.

3. Zusammensetzung nach Anspruch 2, bei der wenigstens 90% des genannten Eisenoxid-Bestandteils in Form von Teilchen vorliegen, die in einem Größenbereich von etwa 0,1 bis 0,4 µm liegen.

4. Zusammensetzung nach irgendeinem der Ansprüche 1 bis 3, bei der die Kaliumverbindung, die zur Formulierung des Katalysators verwendet wird, Kaliumcarbonat ist, das von etwa 20 bis 70 Gew.-% der Zusammensetzung bildet, und bei der das Molverhältnis von $K_2CO_3$ zu $Fe_2O_3$ in Bereich von etwa 0,8:1 bis 1,5:1 liegt.

5. Zusammensetzung nach irgendeinem der Ansprüche 1 bis 4, die zusätzlich bis zu etwa 10 Gew.-% eines Bindemittel-/Füllstoff-Bestandteils enthält, der aus der Gruppe ausgewählt ist, die aus Portlandzement, Calciumaluminat, Sägemehl, Kohlenstoff, Holzmehl, Graphit, Methylzellulose und deren Mischungen besteht.

6. Verfahren zur Dehydrierung von p-Ethyltoluol unter selektiver Bildung von p-Methylstyrol, wobei dieses Verfahren das Kontaktieren von p-Ethyltoluol unter Dehydrier-Reaktionsbedingungen mit einer Katalysatorzusammensetzung umfaßt, die umfaßt:

a) von etwa 30 bis 60 Gew.-% Eisenoxid, berechnet als Eisen(III)oxid;

b) von etwa 13 bis 48 Gew.-% einer Kaliumverbindung, berechnet als Kaliumoxid; und

c) von 0% bis etwa 5% einer Chromverbindung, berechnet als Chrom(III)oxid;

wobei wenigstens 50 Gew.-% des genannten Eisenoxids in der genannten Zusammensetzung in der Form von Teilchen vorliegt, die in einem Größenbereich von etwa 0,05 bis 0,5 µm liegen.

7. Verfahren nach Anspruch 6, bei dem die genannten Dehydrier-Bedingungen eine Temperatur von etwa 500°C bis 700°C und eine stündliche Flüssigkeits-Raumgeschwindigkeit von etwa 0,3 bis 2,0h$^{-1}$ umfassen.

8. Verfahren nach Anspruch 6 oder 7, bei dem p-Ethyltoluol mit dem genannten Katalysator in Gegenwart von Dampf kontaktiert wird und bei dem das Gewichsverhältnis von Dampf zu Kohlenwasserstoff in dem Reaktanen-Einsatzprodukt im Bereich von etwa 1,3:1 bis 4:1 liegt.

9. Verfahren nach irgendeinem der Ansprüche 6 bis 8, bei dem die zur Formulierung der genannten Katalysatorzusammensetzung verwendete Kaliumverbindung Kaliumcarbonat ist, das von etwa 20 bis 70 Gew.-% der Zusammensetzung bildet, und bei dem das Molverhältnis von $K_2CO_3$ zu $Fe_2O_3$ in der genannten Zusammensetzung im Bereich von etwa 0,8:1 bis 1,5:1 liegt.

10. Verfahren nach irgendeinem der Ansprüche 6 bis 9, bei dem die genannte Katalysator-Zusammensetzung zusätzlich bis zu etwa 10 Gew.-% eines Bindemittel-/Füllstoff-Bestandteils enthält, der ausgewählt ist aus der Gruppe, die aus Portlandzement, Calciumaluminat, Sägemehl, Kohlenstoff, Holzmehl, Graphit, Methylzellulose und deren Mischungen besteht.

## Revendications

1. Une composition de catalyseur de déshydrogénation spécialement adaptée à la déshydrogénation du para-éthyltoluène pour former sélectivement du para-méthylstyrène, ladite composition de catalyseur étant caractérisé en ce qu'elle comprend:

a) environ 30 à 60 % en poids d'oxyde de fer, calculé en oxyde ferrique;

b) environ 13 à 48 % en poids de composé de potassium, calculé en oxyde de potassium; et

c) de 0 à environ 5 % de composé de chrome, calculé en oxyde chromique;

au moins 50 % en poids dudit oxyde de fer est présent dans ladite composition sous la forme de particules dont la taille va de 0,05 à 0,5 µm.

2. Une composition selon la revendication 1, caractérisée en ce que:

a) l'oxyde de fer représente environ 35 à 55 % en poids de la composition;

b) le composé de potassium représente environ 27 à 41 % en poids de la composition; et

c) le composé de chrome représente environ 2 à 4 % en poids de la composition.

3. Une composition selon la revendication 2, caractérisée en ce que au moins 90 % dudit oxyde de fer est présent sous la forme de particules d'une dimension variant de 0,1 à 0,4 µm.

4. Une composition selon l'une quelconque des revendications 1 à 3, caractérisée en ce que le composé de potassium utilisé dans la formule du catalyseur est du carbonate de potassium représentant environ 20 % à 70 % en poids de la composition et en ce que le rapport molaire de $K_2CO_3$ à $Fe_2O_3$ va de 0,8:1 à 1,5:1.

5. Une composition selon l'une quelconque des revendications 1 à 4, caractérisée en ce qu'elle contient, en outre, jusqu'à 10 % en poids d'un agent agglomérant/agent de charge, choisi parmi le ciment de fer, l'aluminate de calcium, la sciure de bois, la charbon, la farine de bois, le graphite, la méthylcellulose et leurs mélanges.

6. Un procédé de déshydrogénation du para-éthyltoluène pour former sélectivement du para-méthylstyrène, ledit procédé étant caractérisé en ce que le para-éthyltoluène est mis en contact, dans des conditions de déshydrogénation, avec une composition de catalyseur comprenant:

a) environ 30 à 60 % en poids d'oxyde de fer, calculé en oxyde ferrique;

b) environ 13 à 48 % en poids de composé de potassium, calculé en oxyde de potassium; et

c) de 0 à environ 5 % de composé de chrome, calculé en oxyde chromique;

au moins 50 % en poids dudit oxyde de fer est présent dans ladite composition sous la forme de particules d'une dimension variant de 0,05 à 0,5 µm.

7. Un procédé selon la revendication 6, caractérisé en ce que lesdites conditions de déshydrogénation comportent une température d'environ 500°C à 700°C, et une vitesse spatiale horaire du liquide d'environ 0,3 à 2,0h$^{-1}$.

8. Un procédé selon la revendication 6 ou 7, caractérisé en ce que le para-éthyltoluène est mis en contact avec ledit catalyseur en présence de vapeur et en ce que le rapport en poids de la vapeur à l'hydrocarbure dans l'alimentation en réactif va de 1,3:1 à 4:1.

9. Un procédé selon l'une quelconque des revendications 6 à 8, caractérisé en ce que le composé de potassium utilisé dans la formule dudit catalyseur est le carbonate de potassium, représentant environ 20 à 70 % en poids de la composition, et en ce que le rapport molaire de $K_2CO_3$ à $Fe_2O_3$ dans ladite composition va de 0,8:1 à 1,5,1.

10. Un procédé selon l'une quelconque des revendications 6 à 9, caractérisé en ce que ladite composition de catalyseur contient en outre jusqu'à 10 % en poids d'un agent agglomérant/agent de charge choisi parmi le ciment de fer, l'aluminate de calcium, la sciure de bois, le charbon, la farine de bois, le graphite, la méthylcellulose et leurs mélanges.